# EUROPEAN PATENT SPECIFICATION

(11) **EP 4 478 976 B1**
(45) Date of publication and mention of the grant of the patent: **11.11.2026**
(21) Application number: 23716990.9
(22) Date of filing: 16.03.2023
(51) Int. Cl.: A61B 18/22, A61B 5/00, A61B 18/26

(54) **TECHNIQUES FOR DETERMINING DISTANCE BETWEEN A FIBER END AND A TARGET**
VERFAHREN ZUR BESTIMMUNG DES ABSTANDS ZWISCHEN EINEM FASERENDE UND EINEM ZIEL
TECHNIQUES POUR DÉTERMINER LA DISTANCE ENTRE UNE EXTRÉMITÉ DE FIBRE ET UNE CIBLE

(30) Priority: 17.03.2022 US 202263320943 P
(43) Date of publication of application: 25.12.2024
(73) Proprietor: Lumenis Ltd., 2069200 Yokneam Ilit (IL)
(72) Inventor: WAISMAN, Tal, 3440617 Haifa (IL); KHACHATUROV, Arkady, 2908226 Kiryat Yam (IL)
(74) Representative: Vossius & Partner Patentanwälte Rechtsanwälte mbB
(86) International application number: PCT/US2023/064588
(87) International publication number: WO 2023/178281

(56) References cited:
- US-A1- 2009 244 547
- US-A1- 2010 228 119
- US-A1- 2011 205 523
- US-A1- 2012 232 821
- US-A1- 2016 038 030
- US-A1- 2017 146 335
- US-A1- 2018 245 907
- US-A1- 2021 038 306

## Description

### BACKGROUND

Introduction of lasers into the medical field and the development of fiber optic technologies that use lasers has opened numerous applications in treatments, diagnostics, therapies, and the like. Such applications range from invasive and non-invasive treatments to endoscopic surgeries and image diagnostics. For instance, in urinary stone treatment, the stones are required to be fragmented into smaller pieces. A technology known as laser lithotripsy may be used for such fragmenting processes, wherein for small to medium sized urinary stones, a rigid or flexible ureteroscope is placed through the urinary tract for illumination and imaging. Simultaneously, an optical fiber is inserted through a working channel of the ureteroscope, to a target location (e.g., to the location where the stone is present in the bladder, ureter, or kidney). The laser is then activated to fragment the stone into smaller pieces or to dust it. In another instance, a laser and optic fiber technology is used in coagulation or ablation treatments. During an ablation treatment, laser light is delivered to the tissue to vaporize the tissue. During a coagulation treatment, laser light is used to induce thermal damage within the tissue. Such ablation treatments may be used for treating various clinical conditions, such as Benign Prostate Hyperplasia (BPH), cancers such as prostate cancer. In this context, the technical teaching provided in US2021/038306 A1 is acknowledged.

### BRIEF SUMMARY

The claimed invention is defined by the appended set of claims. Methods of treatment of a living human tissue by surgery and therapy mentioned hereafter, as such, do not form part of the claimed invention thus defined. This Summary is provided to introduce a selection of concepts in a simplified form that are further described below in the Detailed Description. This Summary is not intended to necessarily identify key features or essential features of the claimed subject matter, nor is it intended as an aid in determining the scope of the claimed subject matter.

In one aspect, the present disclosure relates to an apparatus comprising a laser source, an optical fiber, a detector, and a controller. The optical fiber may have a distal end and be configured to pass laser light from the laser source out of the distal end and to receive reflected laser light into the distal end. The controller may include a processor and memory, the memory comprising instructions that when executed by the processor cause the processor to perform one or more of: generate a ranging beam with the laser source, the ranging beam including a linearly changing wavelength sweep, identify a detection signal generated by a detector based on measurement of a mixture of a reference signal, at least one reflection of the ranging beam off a target, and at least one reflection of the ranging beam off a distal end of an optical fiber, analyze the detection signal to determine a frequency component corresponding to the at least one reflection of the ranging beam off the target and the at least one reflection of the ranging beam off the distal end of the optical fiber, and determine a distance between the distal end of the optical fiber and the target based on the frequency component.

In some embodiments, the laser source comprises a first laser source and the instructions, when executed by the processor, further cause the processor to select a mode of operation for a second laser source based on the distance between the distal end of the optical fiber and the target. In various embodiments, the laser source comprises a first laser source and the instructions, when executed by the processor, further cause the processor to generate a treatment beam with a second laser source when the distance between the distal end of the optical fiber and the target is within a threshold distance. In many embodiments, the laser source comprises a first laser source and the instructions, when executed by the processor, further cause the processor to cease generation of the treatment beam with the second laser source when the distance between the distal end of the optical fiber and the target exceeds the threshold distance. In several embodiments, the instructions, when executed by the processor, further cause the processor to generate one or more of an audible, a tactile, and a visual alert when the distance between the distal end of the optical fiber and the target exceeds a threshold distance. In various embodiments, the detection signal is generated by the detector based on measurement of the at least one reflection of the ranging beam off the target, the at least one reflection of the ranging beam off the distal end of the optical fiber, and at least one reflection of the ranging beam off a proximal end of the optical fiber. In various such embodiments, the frequency component comprises a first frequency component and the instructions, when executed by the processor, further cause the processor to analyze the detection signal to determine second and third frequency components, the second frequency component corresponding to the at least one reflection of the ranging beam off the distal end of the optical fiber and the at least one reflection of the ranging beam off the proximal end of the optical fiber and the third frequency component corresponding to the at least one reflection of the ranging beam off the target and the at least one reflection of the ranging beam off the proximal end of the optical fiber. In further such embodiments, the instructions, when executed by the processor, further cause the processor to determine the first frequency component corresponds to the distance the at least one reflection of the ranging beam off the target and the at least one reflection of the ranging beam off the distal end of the optical fiber based on the first frequency component being higher than the second and third frequency components. Some embodiments include a filter configured to remove frequencies below a threshold corresponding to reflections of the ranging beam off a proximal end of the optical fiber. Many embodiments include a bandpass filter configured to remove frequencies above a first threshold and below a second threshold.

In another aspect, the present disclosure relates to at least one non-transitory computer-readable medium comprising a set of instructions that, in response to being executed by a processor circuit, cause the processor circuit to perform one or more of: generate a ranging beam with a laser source, the ranging beam including a linearly changing wavelength sweep; identify a detection signal generated by a detector based on measurement of a mixture of a reference signal, at least one reflection of the ranging beam off a target, and at least one reflection of the ranging beam off a distal end of an optical fiber; analyze the detection signal to determine a frequency component corresponding to the at least one reflection of the ranging beam off the target and the at least one reflection of the ranging beam off the distal end of the optical fiber; and determine a distance between the distal end of the optical fiber and the target based on the frequency component.

In various embodiments, the set of instructions, in response to execution by the processor circuit, further cause the processor circuit to generate one or more of an audible, a tactile, and a visual alert when the distance between the distal end of the optical fiber and the target exceeds a threshold distance. In some embodiments, the set of instructions, in response to execution by the processor circuit, further cause the processor circuit to perform a Fourier analysis on the detection signal to determine the frequency component. In many embodiments, the set of instructions, in response to execution by the processor circuit, further cause the processor circuit to filter out at least a portion of the detection signal based on a reflection frequency associated with a proximal end of the optical fiber.

In yet another aspect, the present disclosure relates to a system comprising a laser source, an optical fiber, a detector, and a controller. The optical fiber may have a distal end and be configured to pass laser light from the laser source out of the distal end and to receive reflected laser light into the distal end. The controller may include a processor and memory, the memory comprising instructions that when executed by the processor cause the processor to perform one or more of: generate a ranging beam with the laser source, the ranging beam including a linearly changing wavelength sweep, identify a detection signal generated by a detector based on measurement of a mixture of at least one reflection of the ranging beam off a target and at least one reflection of the ranging beam off a distal end of an optical fiber, analyze the detection signal to determine first and second frequency components, the first frequency component corresponding to the at least one reflection of the ranging beam off the target and the second frequency component corresponding to the at least one reflection of the ranging beam off the distal end of the optical fiber, and determine a distance between the distal end of the optical fiber and the target based on the first and second frequency components.

Some embodiments include a beam splitter configured to direct a portion of the ranging beam toward the detector, and wherein the detection signal is generated by the detector based on measurement of the portion of the ranging beam, the at least one reflection of the ranging beam off a target, and the at least one reflection of the ranging beam off the distal end of the optical fiber. In some such embodiments, measurement of the portion of the ranging beam, the at least one reflection of the ranging beam off a target, and the at least one reflection of the ranging beam off the distal end of the optical fiber comprises measurement of an interference pattern created on the detector. Various embodiments include a bandpass filter configured to remove frequencies above a first threshold and below a second threshold. In many embodiments, the detector comprises a PIN photodiode or an avalanche photodiode. In several embodiments, the laser source comprises a diode laser, wherein a current of the diode laser is varied to produce the linearly changing wavelength sweep of the ranging beam.

### BRIEF DESCRIPTION OF THE DRAWINGS

Non-limiting embodiments of the present disclosure are described by way of example with reference to the accompanying figures, which are schematic and not intended to be drawn to scale. In will be appreciated that various figures included in this disclosure may omit some components, illustrate portions of some components, and/or present some components as transparent to facilitate illustration and description of components that may otherwise appear hidden. For purposes of clarity, not every component is labelled in every figure, nor is every component of each embodiment shown where illustration is not necessary to allow those of ordinary skill in the art to understand the disclosure. In the figures:
FIG. 1 illustrates a block diagram of an exemplary laser system according to one or more embodiments described hereby.
FIG. 2 illustrates various aspects of an exemplary measurement laser sub-system according to one or more embodiments described hereby.
FIG. 3 illustrates various aspects of an additional (simpler) exemplary measurement laser system according to one or more embodiments described hereby.
FIG. 4 illustrates various aspects of target reflections according to one or more embodiments described hereby.
FIG. 5A illustrates various aspects of theoretical reflection frequencies according to one or more embodiments described hereby.
FIG. 5B illustrates various aspects of measured reflection frequencies according to one or more embodiments described hereby.
FIG. 6 illustrates an exemplary logic flow according to one or more embodiments described hereby.
FIG. 7 illustrates an exemplary logic flow according to one or more embodiments described hereby.
FIG. 8 illustrates a block diagram of an exemplary computer system for implementing embodiments consistent with the present disclosure.

### DETAILED DESCRIPTION

The present disclosure provides a method and system for estimating the distance between an optical fiber end and a target. Treatments which use laser and optic fiber technology need to ensure that the laser is at the appropriate position and settings with respect to a target (stone, tissue, tumor etc.) to achieve the clinical objective of tissue ablation, coagulation, stone fragmentation, dusting, and the like. Accordingly, it is important to know the distance between the target and end of the optical fiber (distal end) where the laser light is emitted, since the laser treatment parameters, such as energy, pulse width, laser power modulation, and/or repetition rate, are often determined based on the distance between the tip of the optical fiber to the target.

Further, the efficiency of treatments using lasers often depend upon the relative position and orientation of the optical fiber tip with respect to the target. However, due to various factors such as movement of the optical fiber with respect to position and orientation within the body of a subject (for instance, a patient), tissue environment, movement of the tissue or stone, surface of the target, color of the target, pigment of the target, optical fiber tip degradation during a treatment, water irrigation, and turbid environment (e.g., due to dusting), and the like, it is extremely difficult to determine or estimate the distance between the optical fiber tip and the target. Determining the distance between the optical fiber tip and the target is further complicated by the fact that the optical fiber tip is typically inserted into the body of the subject.

Incorrect estimation of the distance between the fiber end and the target and incorrect estimation of the orientation of the fiber end can lead to aiming the laser at a region which is not the region of interest of the target. This may lead to unnecessary complications, and in some cases, it can also lead to permanent damage to certain parts of the tissues, organs, etcetera of the subject, which could make portions of the body of the subject dysfunctional. In some other scenarios, incorrect distance measurement and orientation may lead to an increase in the duration of the treatment or may lead to low quality ablation/fragmentation results. In some cases, such as BPH or cancer, if the tumor is not ablated properly, it may lead to regrowth of the tumor (or other undesired tissue) leading to further complications. Therefore, it is important to determine an accurate (or maintain a desired) distance between the optical fiber tip and the target while performing certain treatments using laser and optical fiber technology as discussed above.

One technique to estimate the distance between the distal end of an optical fiber and a target provides for measuring and comparing intensity values of reflections of the light beams, where the light beams are transmitted through the optical fiber by modulating the numerical apertures of the light beams. However, it is not always convenient to shift the numerical apertures of the light beams. Moreover, separation of the reflection of light beams of different numerical apertures, required for these techniques is difficult.

Accordingly, the current disclosure provides techniques for determining distance between a fiber end and a target based on frequency analysis of one or more reflected signals in an efficient and improved manner. In various embodiments, the distance estimation may be utilized for a variety of purposes. For example, the distance may be utilized to provide information and/or warnings to an operator. In one embodiment, a significantly wrong distance may be utilized to provide an indication to an operator that an incorrect object is being targeted. In another example, the distance may be utilized to enable automatic operation of the laser, such as by using distance (and/or other characteristics derived based on reflections) to determine whether a target is present. In one embodiment, a significantly wrong distance may be utilized as an indication that an incorrect object is being targeted, resulting in a protective action being automatically taken (e.g., stop operation of the laser). In yet another example, the distance may be utilized to select a mode of operation for a laser, such as between long-distance and short-distance modes. For example, the threshold distance may be approximately 3mm when using a Thulium laser to generate a treatment beam and approximately 2mm when using a Holmium laser to generate a treatment beam. Additionally, or alternatively, in many embodiments, reflected signals may be utilized to monitor and/or determine the state or condition of one or more components of the laser system. For example, the condition of an optical fiber may be determined based on frequency analysis of one or more reflected signals.

The foregoing has broadly outlined the features and technical advantages of the present disclosure such that the following detailed description of the disclosure may be better understood. It is to be appreciated by those skilled in the art that the embodiments disclosed may be readily utilized as a basis for modifying or designing other structures for carrying out the same purposes of the present disclosure. For instance, aspects and components disclosed hereby may be selectively combined without departing from the scope of this disclosure. The novel features of the disclosure, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present disclosure. Further, although various embodiments may be described with respect to ablation treatments used for treating conditions including Benign Prostate Hyperplasia (BPH) and prostate cancer, reference to these conditions should not be construed as limiting the possible applications of the disclosed aspects. For example, the disclosed aspects may be utilized for treating clinical conditions, cancers (e.g., liver cancer, lung cancer, and the like), and cardiac conditions (e.g., by ablating and/or coagulating a part of the tissue in the heart).

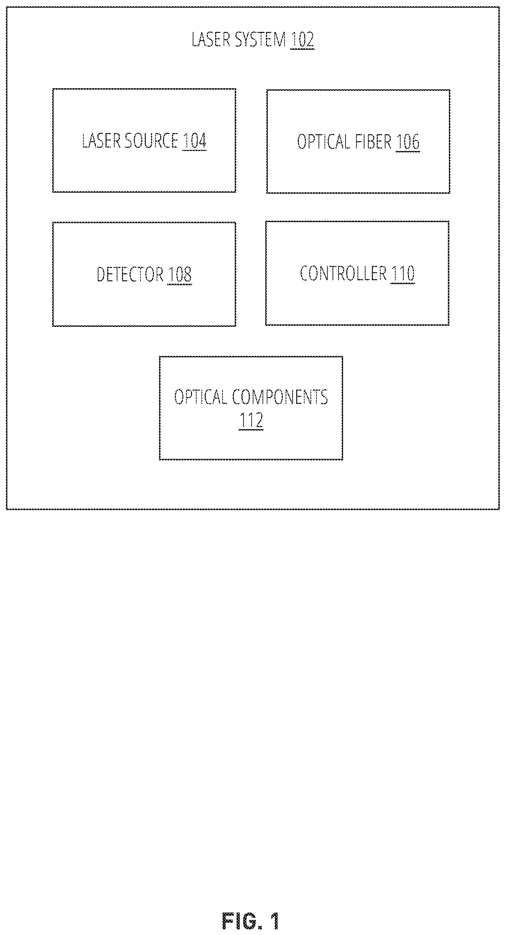

**FIG. 1** illustrates a block diagram of a laser system 102 according to one or more embodiments described hereby. In the illustrated embodiment, laser system 102 includes a laser source 104, an optical fiber 106, a detector 108, a controller 110, and one or more optical components 112. Generally, the laser system 102 may operate to determine a distance between a distal end of the optical fiber 106 and a target, such as with a frequency modulated continuous wave (FMCW) technique. For example, the laser source 104 may have a tunable wavelength and be used to generate a ranging beam with a linearly changing wavelength sweep. The ranging beam may pass through and out of the optical fiber 106, a portion of the ranging beam will be reflected of the fiber tip, and another portion may reflect off a target, enter back into the optical fiber 106 as a return signal, and be directed onto detector 108 for measurement. The detector 108 may generate a detection signal based on measurement of the return signal. The controller 110 may then utilize the detection signal to perform a frequency analysis on the return signal to determine a distance between the optical fiber 106 and the target. For example, the controller 110 may perform a Fourier analysis, such as a fast Fourier transform (FFT), on the return signal. As will be discussed in more detail below, the return signal may comprise reflections from a variety of sources, such as proximal and distal ends of the optical fiber 106 as well as from a target.

Although not illustrated, it will be appreciated that the laser system 102 may be included in, integrated into, or connected to a treatment laser system. The laser system 102 may utilize the distance to deliver laser energy effectively and efficiently to the target. In some examples, laser system 102 may automatically adjust characteristics (e.g., pulse width, power, etc.) of the laser energy generated by the laser source 104 (or a separate treatment laser source) based on the distance, while in other instances laser system 102 may provide an indication (e.g., via a graphical user interface, etc.) of the distance to a user (e.g., physician, technician, etc.) of the laser system 102.

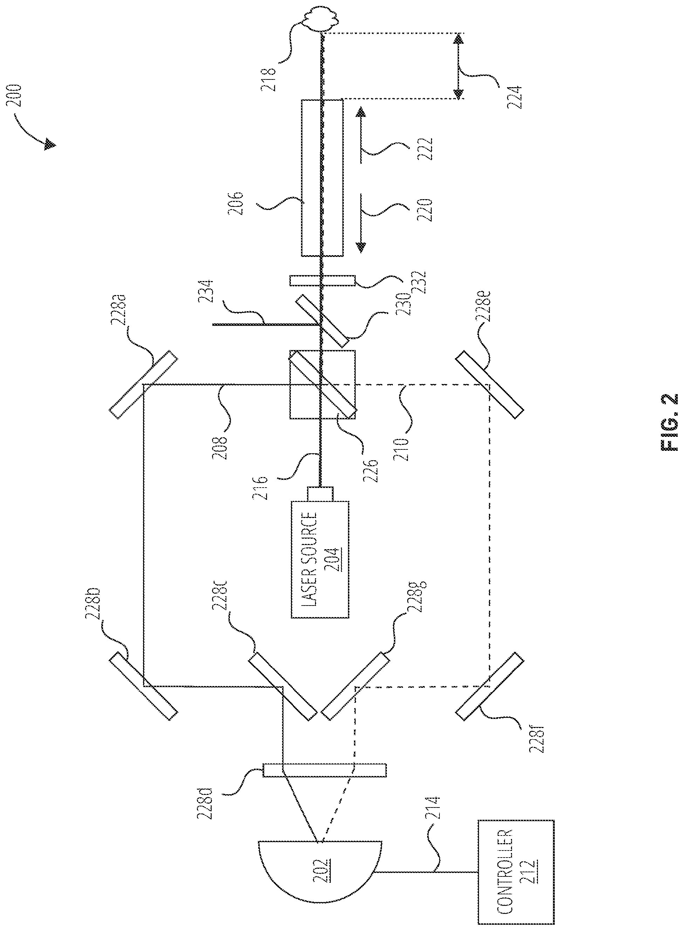

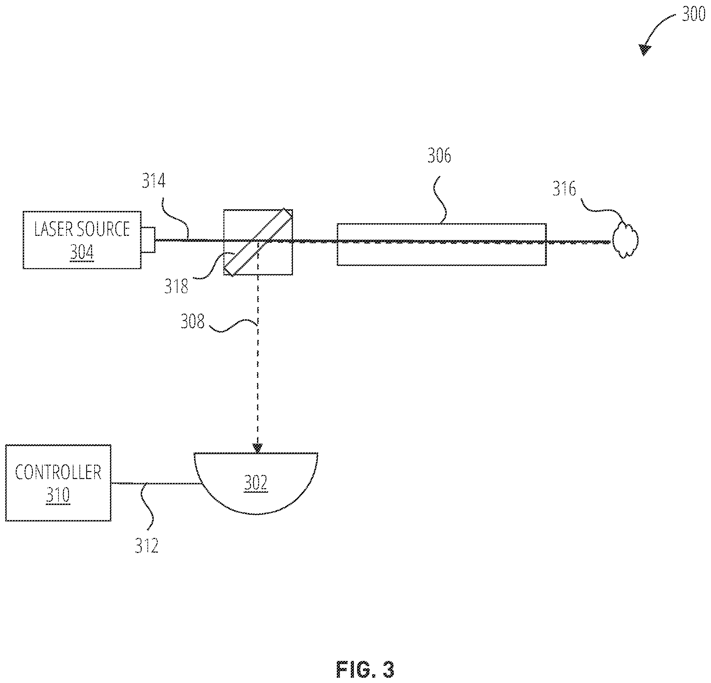

As will be described in more detail below, in some embodiments, such as the embodiment of FIG. 2, a portion of the ranging beam m2ay be redirected and mixed with the return signal for measurement by the detector 108. In other embodiments, such as the embodiment of FIG. 3, a reference signal may not be mixed with the return signal for measurement by the detector 108. Additionally, as will be discussed in more detail below, the return signal may be digitally and/or analogly filtered to remove signal components associated with undesired reflections, such as from a proximal end of the optical fiber 106. In various embodiments, the optical components 112 may be utilized for one or more of splitting, combining, and directing the various signal (e.g., light) components, such as by directing a part of the laser source 104 to the detector 108 for mixing.

The laser source 104 may comprise a wavelength or frequency tunable laser capable of generating a linearly changing wavelength sweep. For example, laser source 104 may include a diode laser and the wavelength may be varied by changing the current of the diode laser. However, it will be appreciated that any technique of causing laser wavelength to change may be used without departing from the scope of this disclosure. In one embodiment, the laser source 104 may include a wavelength tunable vertical cavity surface emitting laser (VCSEL). In some embodiments, the laser source 104 may be utilized to generate, in addition to the ranging beam, a treatment beam to deliver laser energy to the target. However, it will be appreciated that the laser system 102 may include, such as in optical components 112, one or more laser sources different than laser source 104 to generate the treatment beam to deliver laser energy to the target. To this end, optical components 112 may include one or more devices utilized to realize embodiments described hereby. For example, optical components 112 may include, but are not limited to, one or more of laser sources, polarizers, beam splitters, beam combiners, light detectors, filters, wavelength division multiplexers, collimators, circulators, that are configured in various combinations.

More generally, laser light sources, or laser sources, are configured to generate laser light beams with specific and/or varying characteristics (e.g., intensities, wavelengths, etcetera) based on the application. In some embodiments, each laser light source may be designated with a different purpose, for instance, laser source 104 may generate a ranging beam with a linearly changing wavelength sweep, a second laser source may generate a low intensity beam for the purpose of aiming a treatment beam, and a third laser source may generate a high intensity treatment beam for delivering laser energy to the target. Further, each laser light source may have the same aperture or different apertures. Additionally, each laser light source may be configured to generate polarized laser light or unpolarized/depolarized light.

Polarizers may include the optical components that act as an optical filter. For example, polarizers may be configured to allow light beams of a specific polarization to pass through, and to block the light beams of different polarizations. Therefore, when undefined light (or light beams of mixed polarity) is provided as input to a polarizer, the polarizer provides a well-defined single polarized light beam as an output.

Beam splitters may include the optical components used to split incident light at a designated ratio into two separate beams. Further, beam splitters may be arranged to manipulate light to be incident at a desired angle of incidence (AOI). Therefore, in many embodiments, a beam splitter can be primarily configured with two parameters, a ratio of separation and an AOI. The ratio of separation comprises the ratio of reflection to transmission (reflection/transmission (R/T) ratio) of the beam splitter. Accordingly, as used herein, if the ratio of separation for a beam splitter is indicated as 50:50, it means that the beam splitter splits the incident light beams in a R/T ratio of 50:50. In other words, the beam splitter splits the incident light beams by changing the incident light by reflecting 50 percent and transmitting the other 50 percent. Further, as an example, if the AOI for the beam splitter is indicated as 45 degrees, it means that for light beam incident on the beam splitter at 45 degrees, some portion of the incident light will be transmitted while the other portion of the incident light will be reflected at 90 degrees. Beam splitters may include, but are not limited to, polarizing beam splitters and non-polarizing beam splitters. Polarizing beam splitters may split incident light based on the S-polarization component and P-polarization component, such as, for example by reflecting the S-polarized component of light and transmitting the P-polarized component of light (or vice-versa). In some embodiments, non-polarizing beam splitters may split incident light beams based on a specific R/T ratio while maintaining the original polarization state of the incident light beams.

Beam combiners may include partial reflectors that combine two or more wavelengths of light, such as by using the principle of transmission and reflection as explained above. In many embodiments, a beam combiner may be a combination of beam splitters and mirrors, which perform the functionality of combining light of two or more wavelengths.

Light detectors, or simply detectors, may include devices that detect and/or measure characteristics of light beams and encode the detected and/or measured characteristics in electrical signals (e.g., a detection signal). For example, light detectors may detect the specific type of light beams (as preconfigured), and convert the light energy associated with the detected light beams into electrical signals. In some embodiments, wavelength division multiplexing may include a technology that combines a number of optical carrier signals onto a single optical fiber while using laser lights of different wavelengths. In various embodiments, the detector 108 may comprise an interferometer or a fast detector, such as a photodiode. For instance, detector 108 may include a photodiode with an intrinsic (i.e., undoped) region between n- and p-doped regions, referred to as a PIN photodiode. In another instance the detector 108 may include an avalanche photodiode.

A collimator may include a device that narrows down light beams. To narrow down the light beam, a collimator may be configured to cause the directions of motion to become more aligned in a specific direction (for example, parallel rays), or to cause the spatial cross section of the beam to become smaller. In many embodiments, a collimator may be used to change diverging light from a point source into a parallel beam.

A circulator may include a multi-port optical device configured to receive and emit light via a predetermined sequence of the multiple ports. For example, a circulator may include a three (or four, or five, etcetera) port optical device designed such that, light entering any one port exits from the next port. In one such example, light entering a first port may exit a second port, light entering the second port may exit a third port, and light entering the third port may exit the first port. Oftentimes circulators may be utilized to allow light beams to travel in only one direction.

It is noted that where optical component described herein list specific parameters, such as, a beam splitter having an R/T ratio of 50:50 and an AOI of 45 degrees, these parameters are provided for general understanding of the concepts disclosed and not to be limiting. As a specific example, a beam splitter could be provided in various embodiments described herein having a different R/T ratio and/or AOI than specified here without departing from the scope of the disclosure and claims. In one such example, an AOI of 40 degrees may be utilized. In another such example an R/T ratio of 90:10 may be utilized.

**FIG. 2** illustrates various aspects of a laser system 200 according to one or more embodiments described hereby. The illustrated embodiment includes laser system 200 and a target 218. The laser system 200 includes a laser source 204, a beam splitter 226, a beam combiner 230, a lens 232, an optical fiber 206 with a proximal end 220 and a distal end 222, a detector 202, a controller 212, and a plurality of additional optical components 228a, 228b, 228c, 228d, 228e, 228f, 228g. The target 218 may be located a distance 224 from the distal end 222 of the optical fiber 206. In various embodiments, the laser system 200 may function to determine the distance 224 between the distal end 222 of the optical fiber 206 and the target 218.

In operation, the laser source 204 may generate a ranging beam 216, beam splitter 226 may redirect a portion of the ranging beam 216 towards the detector 202 as reference signal 208 and pass the remaining portion of the ranging beam 216 towards the proximal end 220 of the optical fiber 206. The combiner 230 may operate to combine a treatment beam 234 with the ranging beam 216. It will be appreciated that a source for the treatment beam is not illustrated for simplicity. The lens 232 may operate to focus the combined beams onto the proximal end 20 of the optical fiber 206. In several embodiments, the controller 212 may control generation of the ranging beam 216 by the laser source 204. In the illustrated embodiment, mirrors 228a, 228b, 228c and lens 228d may work in conjunction with the beam splitter 226 to direct the reference signal 208 onto detector 202. However, as will be appreciated, a variety of configurations may be utilized to direct reference signal 208 onto detector 202 without departing from the scope of this disclosure. The remaining portion of the ranging beam 216 may enter the proximal end 220 of the optical fiber 206 and exit the distal end 222 of the optical fiber 206, with a portion of the ranging beam 216 reflected form the distal end 222, and another portion of the ranging beam 216 encountering the target 218. A portion of the ranging beam 216 encountering the target 218 may be reflected back into the distal end 222 of the optical fiber 206 as the return signal 210. In the illustrated embodiment, mirrors 228e, 228f, 228g and lens 228d may work in conjunction with beam splitter 226 to direct the return signal 210 onto detector 202. However, as will be appreciated, a variety of configurations may be utilized to direct return signal 210 onto detector 202 without departing from the scope of this disclosure.

Accordingly, the reference signal 208 and the return signal 210 may be mixed to create an interference pattern on the detector 202, such as via heterodyne optical mixing. The detector 202 may generate detection signal 214 in response to measuring the mixed reference and return signals 208, 210. In many embodiments, the detection signal 214 may have a frequency proportional to the distance from which the signal was reflected. For example, due to the return signal 210 having traveled additional distance it is delayed relative to the reference signal 208, resulting in a wavelength difference between them. This wavelength difference may be linearly dependent on the distance travelled. The controller 212 may then determine the distance 224 between the distal end 222 of the optical fiber 206 and the target 218 based on the detection signal 214. For example, controller 212 may perform a Fourier analysis on the detection signal 214 to determine the distance 224 between the distal end 222 of the optical fiber 206 and the target 218.

Evaluation of an exemplary return signal will now be described in more detail. The evaluation may be based on the following constants: speed of light in vacuum (c) is approximately 299792458 m/s; the refractive index of low hydroxyl (OH) glass (optical fiber), n_{f}, is approximately 1.48; the speed of light in fiber, c/n_{f}, is approximately 202562471.6 m/s; the refractive index of saline solution (or water), n_{w}, is approximately 1.3347; the speed of light in saline solution (or water), c/n_{w}, is approximately 224614114 m/s. The evaluation may be based on the following assumptions: laser base wavelength, λ_{base}, is approximately 1060 nm; the frequency of the laser base wavelength, ω_{base} (c/λ_{base}), is approximately 2.82823×10¹⁴ Hz; the laser wavelength pulling range is approximately 1060-1065.5nm (approximately a 5.5nm tuning range); the wavelength scan period is approximately 5.5 ms; and the frequency rate, ωᵣₐₜₑ (wavelength scan range / scan period), is approximately 2.65437×10¹⁴ Hz/s. In various embodiments, the frequency rate may be the primary parameter defining the signal after mixing frequencies.

As previously mentioned, the signal falling on the detector, referred to as the photodiode or mixer in the exemplary evaluation, may include a reference signal (i.e., a local oscillator signal) and a return signal comprising the following reflection signals: a fiber proximal end signal, a fiber distal end signal, a target signal, and other reflection source signals. The local oscillator signal, S_{LO}, is defined by Equation 1 as shown below: ${S}_{LO} = {A}_{LO} \times cos \left(\left({ω}_{base}-{ω}_{rate}t\right)t+{φ}_{LO}\right)$ Equation 1

In the local oscillator signal, *A_{LO}* is the amplitude (intensity) of the Local Oscillator (LO) signal that is delivered to the photodiode (mixer). The fiber proximal end signal, S_{FP}, is defined by Equation 2 as shown below: ${S}_{FP} = {A}_{FP} \times cos \left(\left({ω}_{base}-{ω}_{rate}\left(t-Δ{t}_{FP}\right)\right)t+{φ}_{FP}\right)$ Equation 2

In the fiber proximal end signal, *A_{FP}* is the amplitude (intensity) of the fiber proximal (FP) end reflection signal that is delivered to the photodiode (mixer). Based on the fiber glass index of 1.48 to air index, *A_{FP}* = 0.0375. The fiber distal end signal, S_{FD}, is defined by Equation 3 as shown below: ${S}_{FD} = {A}_{FD} \times cos \left(\left({ω}_{base}-{ω}_{rate}\left(t-Δ{t}_{FD}\right)\right)t+{φ}_{FD}\right)$ Equation 3

In the fiber distal end signal, *A_{FD}* is the amplitude (intensity) of the fiber distal (FD) end reflection signal that is delivered to the photodiode (mixer). Based on the fiber glass index of 1.48 to water (saline) index 0f 1.34, and the intensity loss due to reflection of proximal end, *A_{FD}* = 0.0025 × (1- *A_{FP}*) = 0.00237. The target signal, S_{T}, is defined by Equation 4 as shown below: ${S}_{T} = {A}_{T} \times cos \left(\left({ω}_{base}-{ω}_{rate}\left(t-Δ{t}_{T}\right)\right)t+{φ}_{T}\right)$ Equation 4

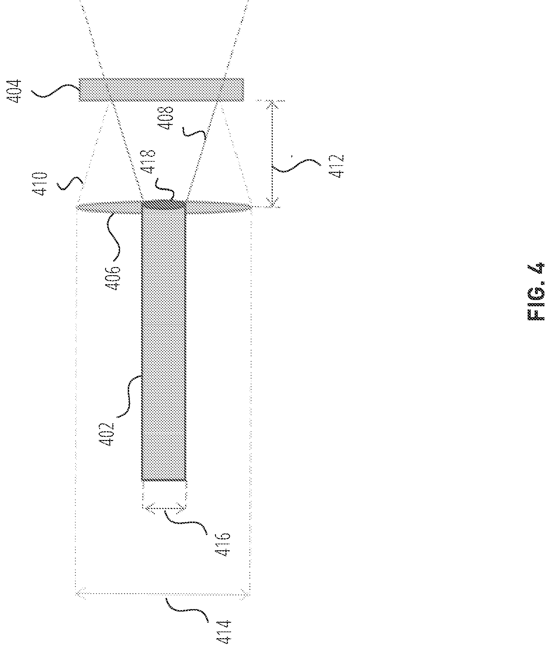

In the target signal, *A_{T}* is the amplitude (intensity) of the target reflection signal that is delivered to the photodiode (mixer). The signal reflected from the target may be calculated as the ratio of areas of the fiber tip versus the area of the conus base created by the beam divergence, times the reflection coefficient of the target. An exemplary calculation of this ratio may proceed as follows with reference to **FIG. 4****,** a portion of a ranging beam 408 reflected as a target reflection 410 from a target 404 can be calculated as the ratio of area of a tip 418 of an optical fiber 402 versus the area of a conus base 406 created by the beam divergence, times the reflection coefficient of the target. For fiber with a numerical aperture (NA) of 0.22, the divergence angle may be 0.166 Rad. Thus, the reflected spot diameter 414, D, from target 404 at a length 412, L, at the tip 418 plane with tip 418 having diameter 416, *d,* is defined by Equation 5 as shown below: $D = 2 \times \left(2L\times 0.166\right) + d$ Equation 5

Thus, when optical fiber 402 has a diameter of 230 micrometers (µm), the above equation reduces to 0.66×L+0.23mm, and the ratio of areas is defined by Equation 6 as shown below: ${\left(d/D\right)}^{2} = {\left(d/\left(0.66\times L+d\right)\right)}^{2}$ Equation 6

Accordingly, as an example, for 230 µm fiber with L=1mm, the ratio is 0.51 and for L=3mm, the ratio is 0.32. The reflectivity, or more correctly, the reflectivity combined with diffusion of light by the target surface is assumed to be in the range of 10%-90%. This means that *A_{T}* is in the range of 0.03 × (1- *A_{FP}*) × *(1- A_{FD}*) to 0.45 × (1- *A_{FP}*) × (1- *A_{FD}*), or 0.03 to 0.43. This means that the target signal is from approximately 12 times to approximately 180 times higher than the signal from the proximal end (45dB maximum difference).

Continuing with the evaluation of an exemplary return signal, signal mixing or detection will now be described. The detector (photodiode) behavior is square law for light intensity (and linear for light power). Therefore, when several light signals are incident on the detector, its output (current) is going to be defined by Equation 7 as shown below: ${I}_{d} = {\left({\sum }_{i} {S}_{i}\right)}^{2}$ Equation 7

Thus, for the signals listed above, the output is going to be defined by Equation 8 as shown below: ${I}_{d =} {\left({S}_{LO}+{S}_{FP}+{S}_{FD}+{S}_{T}\right)}^{2}$ Equation 8

Which equates to Equation 9 as shown below: 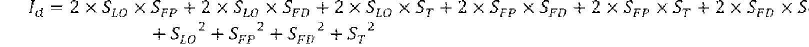 Id = 2 × SLO × SFP + 2 × SLO × SFD + 2 × SLO × ST + 2 × SFP × SFD + 2 × SFP × ST + 2 × SFD × ST + SLO2 +SFP2 + SFD2 + ST2 Equation 9

Further, each component is of a basic type as shown below in Equation 10: ${A}_{1} \times {A}_{2} \times cos \left(\left({ω}_{base}-{ω}_{rate}\left(t-Δ{t}_{1}\right)\right)t+{φ}_{1}\right) \times cos \left(\left({ω}_{base}-{ω}_{rate}\left(t-Δ{t}_{2}\right)\right)t+{φ}_{2}\right)$ Equation 10

Using cosine multiplication formula Equation 10 becomes Equation 11, shown below: $\frac{{A}_{1} {A}_{2}}{2} \left(\begin{matrix}cos \left(\left(2\times {ω}_{base}-{ω}_{rate}\left(2\times t-Δ{t}_{1}-Δ{t}_{2}\right)\right)t+{φ}_{1}+{φ}_{2}\right) \\ + cos \left(\left({ω}_{rate}\left(Δ{t}_{1}-Δ{t}_{2}\right)\right)t+{φ}_{1}-{φ}_{2}\right)\end{matrix}\right)$ Equation 11

Interpreting these general results leads to the following: (1) the sum part of the multiplication result is at base frequency of 2×ω_{base} and will not be sensed by the photodiode; and (2) the difference part of the multiplication will generate a current out of the photodiode at a frequency of *ωᵣₐₜₑ*(*Δt*₁ -Δ*t*₂), which is proportional to the distance difference between the reflectors. Phases may be neglected as well since the measurement is going to be of the frequency (and maybe the amplitude) only.

Therefore, after these assumptions, the detected current is going to be defined by Equation 12 as shown below: ${I}_{d} = \begin{matrix}\frac{{A}_{LO} {A}_{FP}}{2} cos \left(\left({ω}_{rate}\left(Δ{t}_{FP}-Δ{t}_{LO}\right)\right)t\right) + \\ \frac{{A}_{LO} {A}_{FD}}{2} cos \left(\left({ω}_{rate}\left(Δ{t}_{FD}-Δ{t}_{LO}\right)\right)t\right) + \\ \frac{{A}_{LO} {A}_{T}}{2} cos \left(\left({ω}_{rate}\left(Δ{t}_{T}-Δ{t}_{LO}\right)\right)t\right) + \\ \frac{{A}_{FP} {A}_{FD}}{2} cos \left(\left({ω}_{rate}\left(Δ{t}_{FD}-Δ{t}_{FP}\right)\right)t\right) + \\ \frac{{A}_{FP} {A}_{T}}{2} cos \left(\left({ω}_{rate}\left(Δ{t}_{T}-Δ{t}_{FP}\right)\right)t\right) + \\ \frac{{A}_{FD} {A}_{T}}{2} cos \left(\left({ω}_{rate}\left(Δ{t}_{T}-Δ{t}_{FD}\right)\right)t\right) + \\ \frac{{{A}_{LO}}^{2}}{2} + \frac{{{A}_{FP}}^{2}}{2} + \frac{{{A}_{FD}}^{2}}{2} + \frac{{{A}_{T}}^{2}}{2}\end{matrix}$ Equation 12

Accordingly, each of the first six lines in the equation above represents a frequency proportional to the distance difference between the reflectors. The following table, Table 1, shows the resulting frequencies for a fiber of 2.5 m assuming: the propagation in air (from interferometry optics to proximal end) is 0.2 m; the fiber length is 2.5 m; the fiber tip to target distance is 1 mm; the laser wavelength is 1060 nm; the scan range is 5.5 nm; and the scan rate is 5.5 ms.

**Table 1**

| | Local Oscillator | Proximal end frequency [Hz] | Distal end frequency [Hz] | Target frequency [Hz] |
|---|---|---|---|---|
| Local Oscillator | - | 354.16E+03 | 6.9061E+06 | 6.9085E+06 |
| Proximal end frequency | | - | 6.5520E+06 | 6.5543E+06 |
| Distal end frequency | | | - | 2.3635E+03 |
| Target frequency | | | | - |

As shown in Table 1, the signal of interest (Target vs. Distal end frequency difference) is clearly separated in frequency from the rest of the signals. The following table, Table 2, shows results with the fiber length adjusted from 2.5 m to 2 m.

**Table 2**

| | Local Oscillator | Proximal end frequency [Hz] | Distal end frequency [Hz] | Target frequency [Hz] |
|---|---|---|---|---|
| Local Oscillator | - | 354.16E+03 | 5.5957E+06 | 5.5981E+06 |
| Proximal end frequency | | - | 5.2416E+06 | 5.2439E+06 |
| Distal end frequency | | | - | 2.3635E+03 |
| Target frequency | | | | - |

The calculation of amplitudes of the signal for low reflectivity of target (0.03) and assuming local oscillator feedback signal (reference signal) amplitude of 0.01 (1%) is shown in Table 3 below:

**Table 3**

| | Local Oscillator | Proximal end | Distal end | Target |
|---|---|---|---|---|
| Local Oscillator | 0.00005 | 1.8750E-04 | 1.1850E-05 | 1.5000E-04 |
| Proximal end | | 0.0007031 | 2.9625E-05 | 5.6250E-04 |
| Distal end | | | 2.80845E-06 | 3.5550E-05 |
| Target | | | | 0.00045 |

The amplitude of the signal of target to fiber tip is 3.5550×10⁻⁵ for low reflectivity. The direct current (DC) level amplitude (i.e., the sum of all the amplitudes on the diagonal) is 0.001206. The ration between the DC component, and the fiber tip-target components is approximately 34, or 30.6 decibels (dB). The calculation of amplitudes of the signals for high reflectivity of target (0.45) and assuming Local Oscillator feedback signal amplitude of 0.01 (1%) is shown in Table 4 below:

**Table 4**

| | Local Oscillator | Proximal end | Distal end | Target |
|---|---|---|---|---|
| Local Oscillator | 0.00005 | 1.8750E-04 | 1.1850E-05 | 2.2500E-03 |
| Proximal end | | 0.0007031 | 2.9625E-05 | 8.4375E-03 |
| Distal end | | | 2.80845E-06 | 5.3325E-04 |
| Target | | | | 0.10125 |

The amplitude of the signal of target to fiber tip is 5.3325×10⁻⁴ for high reflectivity. The DC level amplitude (the sum of all the amplitudes on the diagonal) is 0.001206. The ratio between the DC component and the fiber tip-target components is approximately 191, or 45.6 dB. As can be seen from the tables, the main contributor to the DC component is the target reflection, in the range of 30-45 dB above the signal. This level is easily resolved at the signal processing level but could also be filtered out using a high pass filter at the analog stage.

Continuing with the evaluation of an exemplary return signal, signal processing and measurement will now be described. In order to measure the frequencies of the reflections, the signal should be sampled at 24Mhz. This will provide 128K points for a Fast Fourier Transform (FFT) calculation, and bin resolution of 366Hz.

For fiber tip - target measurement, the frequency range of approximately 300 Hz to approximately 12,000 Hz (distance of 0.13 - 5mm) should examined, searching for the maximum point. The frequency of the maximum point corresponds to the fiber tip - target distance. This value should also be compared to a threshold, such as 12,000 Hz, which will eliminate the possibility of target reflection not present.

Other reflection points frequencies can also be isolated and found. In this case measuring their amplitude can provide some information regarding the reflecting surface condition for one or more of: proximal end condition, fiber length, distal end condition, target reflectivity (combined with information on target distance), and unwanted reflections indicating a break in the fiber.

Regarding interference from other sources, in principle, all reflecting surfaces at 90° will cause a reflection, and all these reflections will be mixed between them, creating a frequency proportional to the distance between them with an amplitude that is the multiplication of these amplitudes. For example, the lens will cause some small reflection (specular reflection from a single point) from both surfaces, and since these surfaces are approximately 3mm apart, they will create a frequency in the same range as fiber tip - target, resulting in interference with the measurement. This example shows that any 2 surfaces with a distance between them in the area of a few millimeters will cause interfering signal.

However, the signals of interest are going to be centered around the frequency corresponding to the fiber length. These are going to be the signals with the highest frequencies. Thus, the following procedure may be utilized to prevent interfering signals. The spectrum may be scanned from the highest frequency (half the sampling rate) down, identifying the first peak higher than a predetermined threshold. In various embodiments, the predetermined threshold may be determined based on the fiber length. In some embodiments, the fiber length may be determined as the length of the production fiber. In other embodiments, the length may be determined by direct measurement once the fiber is connected and when there is no target reflection (e.g., the fiber tip is in the air).

After the first peak higher than the predetermined threshold is identified, the signal may then be filtered using a bandpass filter around that frequency. More generally, a bandpass filter may remove frequencies from a signal that are above a first threshold and below a second threshold. Next, the remaining signal may be squared, creating frequencies of a difference of the present signals, and of their sum (like the action of the photodiode). Then, a low pass filter may be used on the resulting signal to remove the sum component of the mixed signal. Finally, the frequency of the remaining signal may be estimated, which is directly proportional to the fiber tip-target distance.

An exemplary operating point for laser system 200 may include the following. The laser source 204 may include a direct control tuning laser diode having a wavelength of 1060 nanometers (nm) with a tuning range of 5.5nm and a sweep time of 5.5 milliseconds (ms). For example, laser system 200 may include a pigtailed fiber laser with a wavelength tuning range of 30 or 50 nm and a minimum output power of 0.1 milliwatts (mW). The optical fiber 206 may have a length of 2.5 meters (m) and the distance 224 from the distal end 222 of the optical fiber 206 to the target 218 may be 1 millimeters (mm). Thus, the ranging beam 216 may travel 20 centimeters (cm) in air, 2.5 m through the optical fiber 206, and 1 mm through water to the target 218. These parameters may result in a proximal end reflection of 354 kilohertz (kHz), a distal end reflection of 6.9061 megahertz (MHz), and a target reflection of 6.9085 MHz. Resulting in a frequency difference between the distal end 222 and the target 218 of 2.363 kHz.

In embodiments utilizing a direct sampling approach without mixing the reference signal 208, the sampling rate may be 24 MHz (Nyquist limit of 14MHz) and the controller 212 may perform an 128K (131,072) point FFT with a frequency resolution (FFT bin size) of 366 Hz, resulting in a distance resolution of 0.155mm. In some such embodiments, the processing time for the 128K FFT may be approximately 4 ms.

In other embodiments a super heterodyning approach may be utilized. In such approaches the proximal end reflection measurement may be sacrificed by filtering out, such as with a high pass filter, or processed separately. The filtered signal may be mixed (heterodyned) with a local signal, reducing the frequency of the main signals. The limit of heterodyning frequency may be constrained by the lower signal frequency that must be preserved. For a 2m fiber, the distal end reflection may generate a frequency of 5.7 MHz. Therefore, a local oscillator frequency of 5 MHz may be used. The resulting frequency for distal end / target reflections may become approximately 2 MHz and a sampling rate of 6 MHz may be used. This can enable the FFT to be reduced to 32,768 points, resulting in a reduced computational load over the direct sampling approach.

In some embodiments, the wavelength tuning may be by diode current. However, using current to control the wavelength may result in a limited tuning range when compared to other devices. For example, linearly scanning the current from 100 milliamps (mA) to 450 mA may give a tuning range of 0.4 nm, but to achieve the same frequency change rate of the pigtailed fiber laser described above, the scan time would be 0.4 ms. However, this is too short to sample the necessary number of points for an FFT. Accordingly, in various embodiments, the scan may be repeated and concatenated to achieve the necessary number of points (14 times based on the 0.4 nm and 0.4 ms).

Accordingly, a number of considerations may go into choosing the appropriate laser source. Considerations in selecting a direct control tuning laser configuration may include one or more of: convenient wavelength scan control, constant power throughout the scan, variations in linearity of the scan and power output stability may result in increased Fourier domain line width and reduce distance resolution, and the power output level (e.g., 0.1 mW) may result in weak reflected signals and the need for avalanche photodiode instead of PIN photodiode and/or high amplification. Considerations in selecting a wavelength tuning by diode current laser configuration may include one or more of: pulling the wavelength by changing the current may be a basic characteristic, power level changes during pulse may result in increased Fourier domain line width, high power levels may create an eye safety issue, and the need for much higher operating currents compared to direct control tuning laser configurations. The power level changes during a pulse resulting in increased Fourier domain line width for wavelength tuning by diode current laser configurations may be mitigated by increasing the frequency change rate (faster scanning), which can result in increased fiber tip - target frequency separation. Additionally, the eye safety issue can be mitigated by moving to another wavelength (e.g., from 1060 nm to 1350 nm).

**FIG. 3** illustrates various aspects of a laser system 300 according to one or more embodiments described hereby. The illustrated embodiment includes laser system 300 and a target 316. The laser system 300 includes a laser source 304, a beam splitter 318, an optical fiber 306, a detector 302, and a controller 310. In various embodiments, the laser system 300 may function to determine the distance between a distal end of the optical fiber 306 and the target 316. In operation, the laser source 304 may generate a ranging beam 314 that passes through the beam splitter 318 and through the optical fiber 306 onto the target 316. In several embodiments, the controller 310 may control generation of the ranging beam 314 by the laser source 304. A portion of the ranging beam 314 may be reflected off the fiber distal end and the target 316 and back into the optical fiber 306 as a return signal 308. The return signal 308 may pass through the optical fiber 306 and be directed toward the detector 302 by the beam splitter 318. The detector 302 may generate detection signal 312 based on measurement of the remaining portion of the return signal 308 and controller 310 may perform a frequency analysis on the detection signal 312 to determine the distance between the target 316 and the distal end of the optical fiber 306. It will be appreciated that a variety of configurations may be utilized to direct the ranging beam 314 onto the target 316, filter the return signal 308, and direct the return signal 308 onto the detector 302 without departing from the scope of this disclosure.

The return signal 308 may include a reflection from a variety of sources, such as the proximal and distal ends of the optical fiber 306 as well as the target 316. By mixing all of the signals present at the detector, the detection signal 312 includes a frequency that is the direct result of the mixing between reflections signals from the distal end of the optical fiber 306 and the target 316. More generally, mixing creates signals that are the sum of the original signal frequencies (which is then filtered out) and the difference of the remaining frequencies is measured. Accordingly, using the frequency modulation of the transmitted signal and measuring the frequencies generated by the detector 302 in detection signal 312, controller 310 can detect a frequency proportional to the distance between the distal end of the optical fiber 306 and the target 316. This can be accomplished without mixing in a reference signal as described with respect to laser system 200. Without mixing in the reference signal (which would be a portion of the ranging beam 314), Equation 12 discussed above reduces to Equation 13 as shown below: ${I}_{α} = \begin{matrix}\frac{{A}_{FP} {A}_{FD}}{2} cos \left(\left({ω}_{rate}\left(Δ{t}_{FD}-Δ{t}_{FP}\right)\right)t\right) + \\ \frac{{A}_{FP} {A}_{T}}{2} cos \left(\left({ω}_{rate}\left(Δ{t}_{T}-Δ{t}_{FP}\right)\right)t\right) + \\ \frac{{A}_{FP} {A}_{T}}{2} cos \left(\left({ω}_{rate}\left(Δ{t}_{T}-Δ{t}_{FP}\right)\right)t\right) + \\ \frac{{{A}_{FP}}^{2}}{2} + \frac{{{A}_{FD}}^{2}}{2} + \frac{{{A}_{T}}^{2}}{2}\end{matrix}$ Equation 13

Further, the frequencies of the fiber proximal end to the fiber distal end and the fiber proximal end to the target will be higher than the signal of interest, the fiber distal end to the target, because of the higher distances associated with the fiber proximal end to the fiber distal end and the fiber proximal end to the target. For example, distances associated with fiber proximal end to the fiber distal end and the fiber proximal end to the target will be on the order of 2.5 m while the distance associated with the fiber distal end to the target will be on the order of a few millimeters. Accordingly, the frequencies of the fiber proximal end to the fiber distal end and the fiber proximal end to the target can be readily filtered out, such as using signal processing hardware (analog or digital) and software. However, in the laser system 300 the optical design must assure that no reflections in the measurement system with distances between them of a few millimeters exist at levels comparable to the fiber distal end to the target signal because the reference signal is not being mixed in. This can be achieved by using an optical design in which the reflecting optical surfaces are either placed at an angle, so they do not reflect back to the detector, and/or are coated with appropriate antireflective coatings that reduce such signals.

Further, laser system 300 can be used in cases where the only information required is the fiber tip - target distance. Additionally, laser system 300 may be achieved in a more economical manner than laser system 200. In various embodiments, the laser system may utilize a bandpass filter about the aforementioned first peak frequency higher than a predetermined threshold determined based on length of the fiber. One example may include an analog level bandpass filter for a frequency range of 300 Hz to 24,000 Hz (corresponding to 0.13 mm - 10 mm distances). In such an example, the remaining signal may be sampled with a frequency of 93,750 Hz (1/256 times the original frequency). This will result in 512 samples within the 5 ms sweep time and performing a 512-point FFT will result in the same distance resolution described above with respect to FIG. 2.

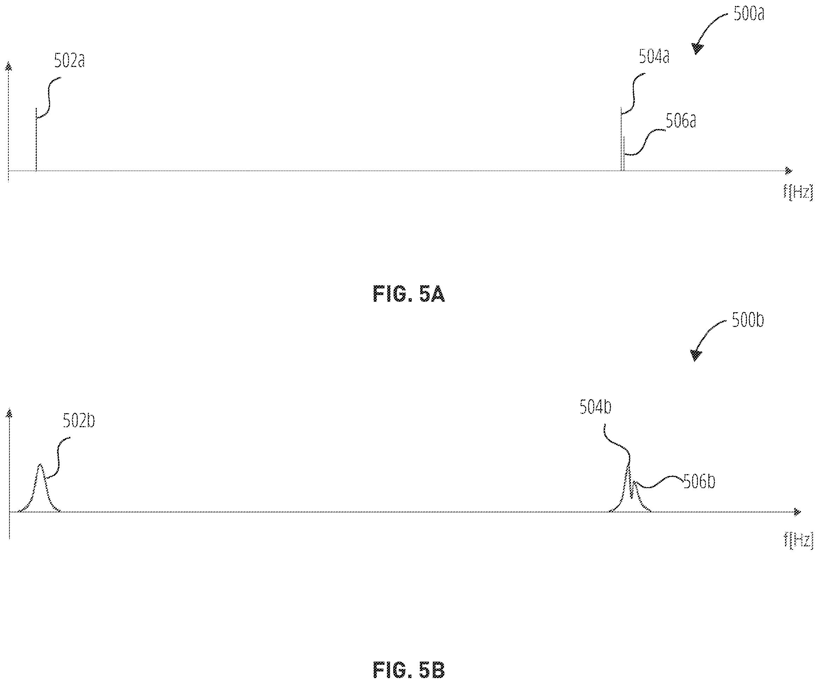

**FIGS. 5A** and **5B** illustrate aspects of reflection frequencies according to one or more embodiments described hereby. More specifically, FIG. 5A illustrates aspects of theoretical reflection frequencies 500a and FIG. 5B illustrates aspects of actual reflection frequencies 500b. Each of the theoretical and actual reflection frequencies 500a, 500b include a proximal fiber reflection 502a, 502b, a distal fiber reflection 504a, 504b, and a target reflection 506a, 506b. The frequency difference between the distal fiber reflections and the target reflections is proportional to the distance between the distal end of the optical fiber and the target. As shown in FIG. 5A, in the theoretical reflection frequencies 500a the values for each reflection are discrete. However, as shown in FIG. 5B, in the actual reflection frequencies 500b the values for each reflection are spread out. This spreading may result from a number of factors, such as one or more of an undesirable amplitude modulation of transmitted light intensity, non-linearity of the frequency modulation, creation of new modes in the multi-mode delivery fiber and their different propagation times. Accordingly, practical distance resolution may be determined by these effects.

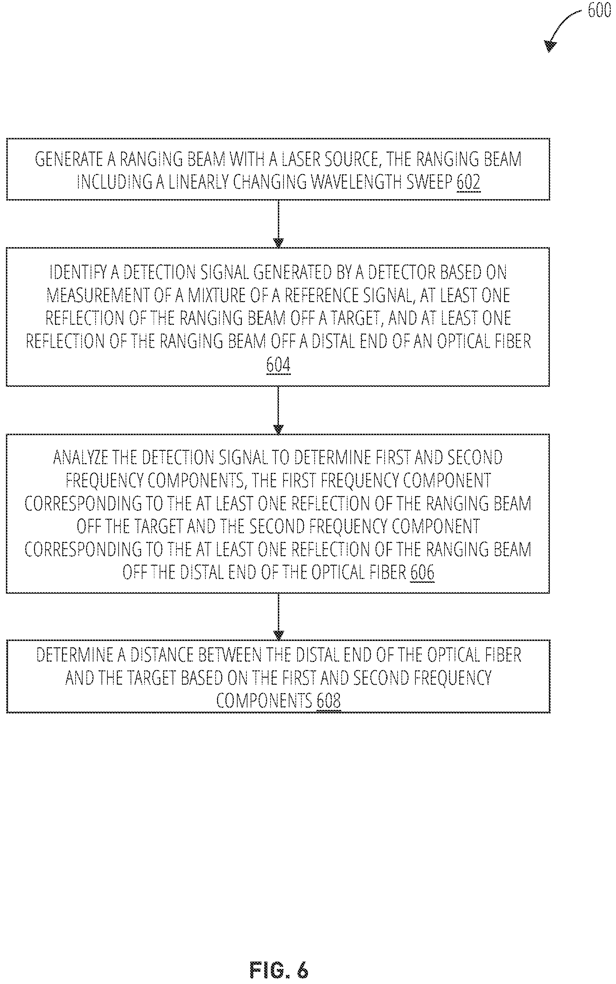

**FIG. 6** illustrates a logic flow 600 for estimating distance between a distal end of an optical fiber and a target according to one or more embodiments described hereby. More specifically, logic flow 600 may be directed to detection that includes mixing done with a reference signal that travels a constant short local path (see e.g., FIG. 2). The logic flow 600 may be representative of some or all of the operations that may be executed by one or more components/devices/systems described hereby, such as one or more portions of laser system 102, laser system 200, and/or laser system 300. The embodiments are not limited in this context.

In the illustrated embodiment, logic flow 600 may begin at block 602. At block 602 "generate a ranging beam with a laser source, the ranging beam including a linearly changing wavelength sweep" a ranging beam with a linearly changing wavelength sweep may be generated by a laser source. For example, laser source 204 may generate ranging beam 216 with a linearly changing wavelength sweep. Continuing to block 604 "identify a detection signal generated by a detector based on measurement of at least one reflection of the ranging beam off a target and at least one reflection of the ranging beam off a distal end of an optical fiber" a detection signal generated by a detector based on measurement of at least one reflection of the ranging beam off a target and at least one reflection of the ranging beam off a distal end of an optical fiber may be identified. For example, controller 212 may identify detection signal 214 generated by detector 202 based, at least in part, on measuring return signal 210 comprising at least one reflection of the ranging beam 216 off of the target 218 and at least one reflection of the ranging beam 216 off the distal end 222 of the optical fiber 206.

At block 606 "analyze the detection signal to determine first and second frequency components, the first frequency component corresponding to the at least one reflection of the ranging beam off the target and the second frequency component corresponding to the at least one reflection of the ranging beam off the distal end of the optical fiber" the detection signal may be analyzed to determine a first frequency component corresponding to the at least one reflection of the ranging beam off the target and a second frequency component corresponding to the at least one reflection of the ranging beam off the distal end of the optical fiber. For example, controller 110 may identify a detection signal comprising distal fiber reflection 504b frequency component corresponding to reflection of the ranging beam off a distal end of the optical fiber 106 and target reflection 506b frequency component corresponding to reflection of the ranging beam off a target. Proceeding to block 608 "determine a distance between the distal end of the optical fiber and the target based on the first and second frequency components" a distance between the distal end of the optical fiber and the target based on the first and second frequency components may be determined. For example, controller 212 may determine distance 224 based on distal fiber reflection 504b and target reflection 506b.

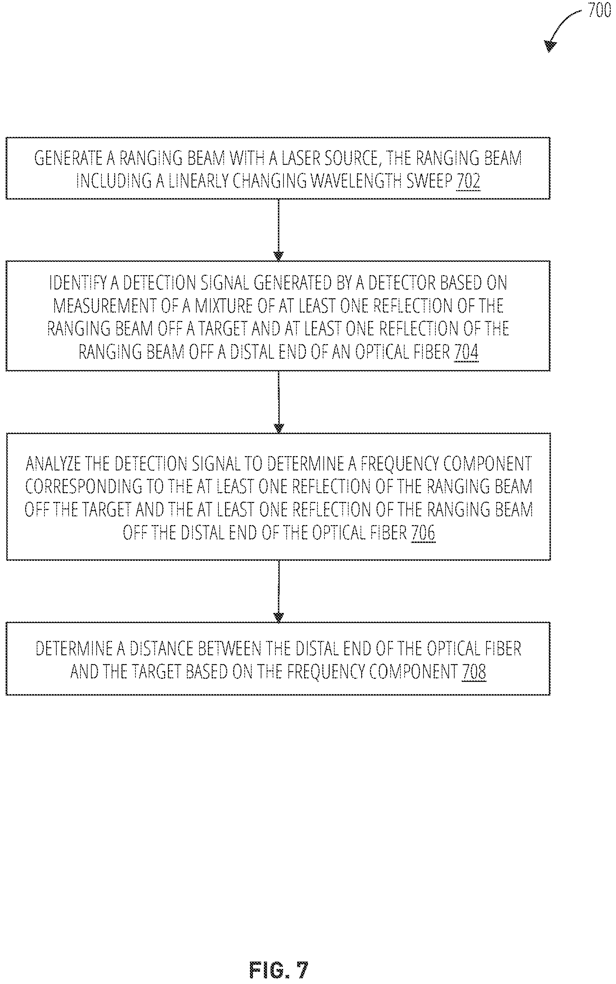

**FIG. 7** illustrates a logic flow 700 for estimating distance between a distal end of an optical fiber and a target according to one or more embodiments described hereby. More specifically, logic flow 700 may be directed to detection that includes a mixing between all signals present on the detector (no reference signal), according to the square law (see e.g., FIG. 3). The logic flow 700 may be representative of some or all of the operations that may be executed by one or more components/devices/systems described hereby, such as one or more portions of laser system 102, laser system 200, and/or laser system 300. The embodiments are not limited in this context.

In the illustrated embodiment, logic flow 700 may begin at block 702. At block 702 "generate a ranging beam with a laser source, the ranging beam including a linearly changing wavelength sweep" a ranging beam with a linearly changing wavelength sweep may be generated by a laser source. For example, laser source 304 may generate ranging beam 314 with a linearly changing wavelength sweep. Continuing to block 704 "identify a detection signal generated by a detector based on measurement of at least one reflection of the ranging beam off a target and at least one reflection of the ranging beam off a distal end of an optical fiber" a detection signal generated by a detector based on measurement of at least one reflection of the ranging beam off a target and at least one reflection of the ranging beam off a distal end of an optical fiber may be identified. For example, controller 310 may identify detection signal 312 generated by detector 302 based, at least in part, on measuring return signal 308 comprising at least one reflection of the ranging beam 314 off of the target 316 and at least one reflection of the ranging beam 314 off a distal end of the optical fiber 306.

At block 706 "analyze the detection signal to determine a frequency component corresponding to the at least one reflection of the ranging beam off the target and the at least one reflection of the ranging beam off the distal end of the optical fiber" the detection signal may be analyzed to determine a frequency component corresponding to the at least one reflection of the ranging beam off the target and the at least one reflection of the ranging beam off the distal end of the optical fiber. For example, controller 310 may identify a frequency component in return signal 308 corresponding to reflection of the ranging beam 314 off a distal end of the optical fiber 306 and reflection of the ranging beam 314 off target 316. Proceeding to block 608 "determine a distance between the distal end of the optical fiber and the target based on the frequency component" a distance between the distal end of the optical fiber and the target based on the first and second frequency components may be determined. For example, controller 310 may determine the distance between a distal end of the optical fiber 306 and the target 316 based on a frequency component in detection signal 312.

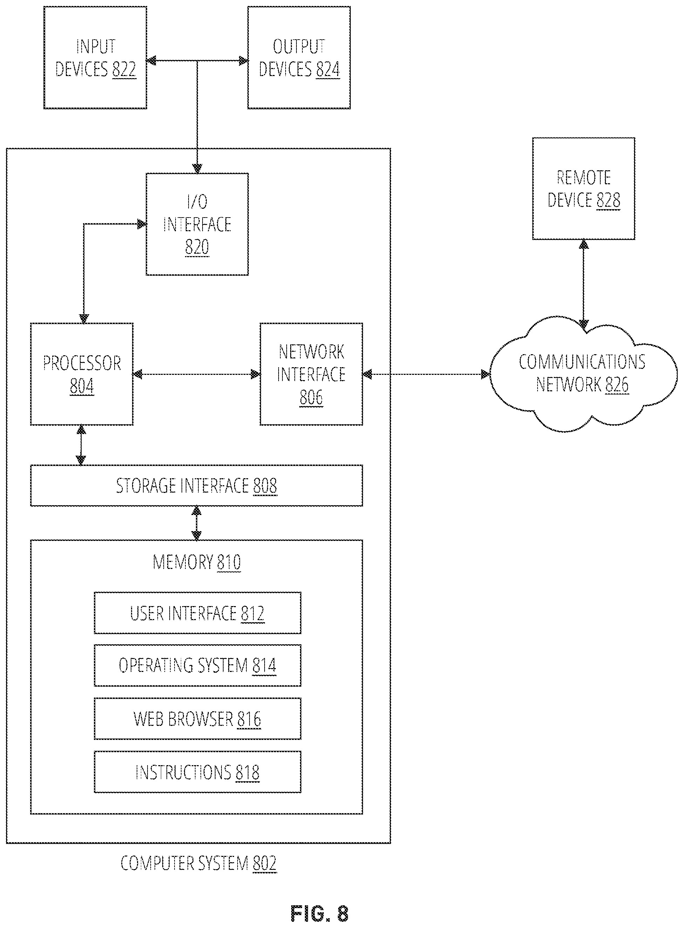

**FIG. 8** is a block diagram of a computer system 802 for implementing embodiments consistent with the present disclosure. In some embodiments, the computer system 802, or one or more portions thereof, may comprise a controller (e.g., controller 110, 212, 310) in a laser system (e.g., laser system 102, 200, 300). Accordingly, in various embodiments, computer system 802 may perform a frequency analysis on a detection signal (e.g., detection signal 214, 312) generated by a detector (e.g., detector 108, 202, 302) based on measurement of one or more signals (e.g., return signal 210, 308 and/or reference signal 208). In various such embodiments, the computer system 802 may be utilized to control operation of the laser system (e.g., laser system 102, 200, 300), such as based on a distance to a target determined based on the frequency analysis. Embodiments are not limited in this context.

The computer system 802 may include a central processing unit ("CPU" or "processor") 804. The processor 804 may include at least one data processor for executing instructions and/or program components for executing user or system-generated processes. A user may include a person, a person using a device such as those included in this disclosure, or such a device itself. The processor 804 may include specialized processing units such as integrated system (bus) controllers, memory management control units, floating point units, graphics processing units, digital signal processing units, etc. The processor 804 may be disposed in communication with input devices 822 and output devices 824 via I/O interface 820. The I/O interface 820 may employ communication protocols/methods such as, without limitation, audio, analog, digital, stereo, IEEE-1394, serial bus, Universal Serial Bus (USB), infrared, PS/2, BNC, coaxial, component, composite, Digital Visual Interface (DVI), high-definition multimedia interface (HDMI), Radio Frequency (RF) antennas, S-Video, Video Graphics Array (VGA), IEEE 802.n /b/g/n/x, Bluetooth, cellular (e.g., Code-Division Multiple Access (CDMA), HighSpeed Packet Access (HSPA+), Global System For Mobile Communications (GSM), Long-Term Evolution (LTE), WiMAX, or the like), etc.

Using the I/O interface 820, computer system 802 may communicate with input devices 822 and output devices 824. In some embodiments, the processor 804 may be disposed in communication with a communications network 826 via a network interface 806. In various embodiments, the communications network 826 may be utilized to communicate with a remote device 828, such as for accessing look-up tables, performing updates, or utilizing external resources. The network interface 806 may communicate with the communications network 826. The network interface 806 may employ connection protocols including, without limitation, direct connect, Ethernet (e.g., twisted pair 10/100/1000 Base T), Transmission Control Protocol/Internet Protocol (TCP/IP), token ring, IEEE 802.11a/b/g/n/x, etc. In some embodiments, one or more portions of the computer system 802 may be integrated into a laser system (e.g., laser system 102, 200, 300). In some such embodiments, one or more components of the laser system may comprise one or more of input devices 822 and/or one or more of output devices 824 (e.g., laser source 104, detector 108, etcetera).

The communications network 826 can be implemented as one of the different types of networks, such as intranet or Local Area Network (LAN), Closed Area Network (CAN) and such. The communications network 826 may either be a dedicated network or a shared network, which represents an association of the different types of networks that use a variety of protocols, for example, Hypertext Transfer Protocol (HTTP), CAN Protocol, Transmission Control Protocol/Internet Protocol (TCP/IP), Wireless Application Protocol (WAP), etc., to communicate with each other. Further, the communications network 826 may include a variety of network devices, including routers, bridges, servers, computing devices, storage devices, etcetera. In some embodiments, the processor 804 may be disposed in communication with a memory 810 (e.g., RAM, ROM, etc. not shown in FIG. 12) via a storage interface 808. The storage interface 808 may connect to memory 810 including, without limitation, memory drives, removable disc drives, etc., employing connection protocols such as Serial Advanced Technology Attachment (SATA), Integrated Drive Electronics (IDE), IEEE-1394, Universal Serial Bus (USB), fiber channel, Small Computer Systems Interface (SCSI), etc. The memory drives may further include a drum, magnetic disc drive, magneto-optical drive, optical drive, Redundant Array of Independent Discs (RAID), solid-state memory devices, solid-state drives, etcetera.

The memory 810 may store a collection of program or database components, including, without limitation, a user interface 812, an operating system 814, a web browser 816, instructions 818, etcetera. In various embodiments, instructions 818 may include instructions that when executed by the processor 804 cause the processor 804 to perform one or more techniques, steps, procedures, and/or methods described herein, such to estimate a distance. For example, instructions to perform logic flow 600 and/or logic flow 700 may be stored in memory 810. In many embodiments, memory 810 includes at least one non-transitory computer-readable medium. In some embodiments, the computer system 802 may store user/application data, such as the data, variables, records, preferences, etc. as described in this disclosure. Such databases may be implemented as fault-tolerant, relational, scalable, secure databases such as Oracle or Sybase.

The operating system 814 may facilitate resource management and operation of the computer system 802. Examples of operating systems include, without limitation, APPLE^{®} MACINTOSH^{®} OS X^{®}, UNIX^{®}, UNIX-like system distributions (E.G., BERKELEY SOFTWARE DISTRIBUTION^{®} (BSD), FREEBSD^{®}, NETBSD^{®}, OPENBSD, etc.), LINUX^{®} DISTRIBUTIONS (E.G., RED HAT^{®}, UBUNTU^{®}, KUBUNTU^{®}, etc.), IBM^{®}OS/2^{®}, MICROSOFT^{®} WINDOWS^{®} (XP^{®}, VISTA^{®}/7/8, 10 etc.), APPLE^{®} IOS^{®}, GOOGLE^{™} ANDROID^{™}, BLACKBERRY^{®} OS, or the like. The user interface 812 may facilitate display, execution, interaction, manipulation, or operation of program components through textual or graphical facilities. For example, user interfaces may provide computer interaction interface elements on a display system operatively connected to the computer system 802, such as cursors, icons, checkboxes, menus, scrollers, windows, widgets, etcetera. Graphical User Interfaces (GUIs) may be employed, including, without limitation, Apple^{®} Macintosh^{®} operating systems' Aqua^{®}, IBM^{®} OS/2^{®}, Microsoft^{®} Windows^{®} (e.g., Aero, Metro, etc.), web interface libraries (e.g., ActiveX^{®}, Java^{®}, JavaScript^{®}, AJAX, HTML, Adobe^{®} Flash^{®}, etcetera), or the like. In some embodiments, the user interface 812 may be integrated with the display and/or user interface of an endoscope.

In some embodiments, the computer system 802 may implement the web browser 816 stored program components. The web browser 816 may be a hypertext viewing application, such as MICROSOFT^{®} INTERNET EXPLORER^{®}, GOOGLE^{™} CHROME^{™}, MOZILLA^{®} FIREFOX^{®}, APPLE^{®} SAFARI^{®}, etcetera. Secure web browsing may be provided using Secure Hypertext Transport Protocol (HTTPS), Secure Sockets Layer (SSL), Transport Layer Security (TLS), etcetera. Web browser 816 may utilize facilities such as AJAX, DHTML, ADOBE^{®} FLASH^{®}, JAVASCRIPT^{®}, JAVA^{®}, Application Programming Interfaces (APIs), etcetera. In some embodiments, the computer system 802 may implement a mail server stored program component. The mail server may be an Internet mail server such as Microsoft Exchange, or the like. The mail server may utilize facilities such as Active Server Pages (ASP), ACTIVEX^{®}, ANSI^{®} C++/C#, MICROSOFT^{®}, .NET, CGI SCRIPTS, JAVA^{®}, JAVASCRIPT^{®}, PERL^{®}, PHP, PYTHON^{®}, WEBOBJECTS^{®}, etcetera. The mail server may utilize communication protocols such as Internet Message Access Protocol (IMAP), Messaging Application Programming Interface (MAPI), MICROSOFT^{®} exchange, Post Office Protocol (POP), Simple Mail Transfer Protocol (SMTP), or the like. In some embodiments, the computer system 802 may implement a mail client stored program component. The mail client may be a mail viewing application, such as APPLE^{®} MAIL, MICROSOFT^{®} ENTOURAGE^{®}, MICROSOFT^{®} OUTLOOK^{®}, MOZILLA^{®} THUNDERBIRD^{®}, etcetera.

Furthermore, memory 810 may include one or more computer-readable storage media utilized in implementing embodiments consistent with the present disclosure. Generally, a computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., non-transitory. Examples include Random Access Memory (RAM), Read-Only Memory (ROM), volatile memory, non-volatile memory, hard drives, Compact Disc (CD) ROMs, Digital Video Disc (DVDs), flash drives, disks, and any other known physical storage media.

In various embodiments, the present disclosure may provide a variety of technical effects and improvements. For example, the present disclosure may enable estimation of distance between a distal end of an optical fiber and a target, by performing a frequency analysis of reflected or return signals. Estimation of the distance based on frequency analysis can provide robustness with respect to different types of targets, target compositions, target colors, target surfaces and the like. The frequency analysis-based techniques and systems disclosed in the present disclosure can be provided to estimate a distance between the distal end of an optical fiber and a target and can facilitate an accurate estimation of the distance. Further, the present disclosure provides processes of estimation of a distance between a distal end of an optical fiber and a target for various types of targets and can provide for estimation of the distance for more and more varied target than conventionally possible. Accordingly, the present disclosure provides systems and methods to more accurately aiming at a target than conventionally possible. More accurate aiming can eliminate or reduce ablating and/or fragmenting incorrect portions of the target (or nontargeted areas, such as healthy tissue), which can lead to adverse outcomes and/or permanent damages. Also, more accurate aiming consumes less time in ablating and/or fragmenting the target, leading to a more efficient system.

In several embodiments, the present disclosure may be used to accurately position and/or aim a treatment beam, such as in low-visibility environments (e.g., environments including dust or target debris). For example, during treatment of a target (e.g., kidney stones) water may get turbid due to the presence of stone fragments or dust. This may reduce (or prevent) the ability to see the target (e.g., the kidney stone). In such scenarios, the present disclosure provides a system to accurately recognize and inform the treating physician about placement of the optical fiber (e.g., whether the fiber is placed in front of the target or whether there is no target detected).

Further, in many embodiments, the present disclosure may be used for distance measurement. For example, the target (e.g., kidney stone) may move around during treatment, which may lead to laser light associated with a treatment beam being incident on unwanted areas (e.g., healthy tissue, or the like) as opposed to being incident on the target. Therefore, the present disclosure may enable automatic and real-time monitoring of the distance between the optical fiber and the target, which in turn can reduce, or eliminate, the possibility of lasing unwanted areas.

Still further, in various embodiments, the present disclosure may be used for the purpose of controlling and/or adjusting one or more operational parameters. For example, during the treatment, the target may move back and forth, or may change its shape and size. Therefore, parameters pre-set for the laser sources before initiating lasing on the target, may become less effective. Conventionally, such pre-set parameters are manually changed which may be error prone and time consuming, or in some cases the pre-set parameters may be left unchanged which may lead to scenarios where the optical fiber may be too close or too far from the target. Therefore, the automatic and real-time monitoring of the distance between the optical fiber and the target, as disclosed in the present disclosure, can enable automatically changing the lasing pre-set parameters to adjust the lasing in accordance with the target shape, position, etcetera for best results.

With respect to the use of substantially any plural and/or singular terms herein, those having skill in the art can translate from the plural to the singular and/or from the singular to the plural as is appropriate to the context and/or application. The various singular and/or plural permutations are expressly set forth herein for sake of clarity and not limitation.

It will be understood by those within the art that, in general, terms used herein, and are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended. For example, as an aid to understanding, the detail description may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to disclosures containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean *at least* the recited number (e.g., the bare recitation of "two recitations," without other modifiers, typically means *at least* two recitations, or *two or more* recitations).

All of the devices and/or methods disclosed and claimed herein can be made and executed without undue experimentation in light of the present disclosure. While the devices and methods of this disclosure have been described in terms of preferred embodiments, it may be apparent to those of skill in the art that variations can be applied to the devices and/or methods and in the steps or in the sequence of steps of the method described herein without departing from the scope of the disclosure. All such similar substitutes and modifications apparent to those skilled in the art are deemed to be within the scope and concept of the disclosure as defined by the appended claims.

## Claims

1. An apparatus, comprising:
a laser source (104, 204, 304) comprising a first laser source;
a second laser source;
an optical fiber (106, 206, 306, 402) having a distal end (222), the optical fiber configured to pass laser light from the laser source out of the distal end and to receive reflected laser light into the distal end;
a detector (108, 202, 302); and
a controller (110, 212, 310) comprising a processor (804) and memory (810), the memory comprising instructions that when executed by the processor cause the processor to:
generate (602) a ranging beam (216, 314, 408) with the laser source, the ranging beam including a linearly changing wavelength sweep,
identify (604) a detection signal (214, 312) generated by a detector based on measurement of a mixture of a reference signal (208), at least one reflection (410) of the ranging beam off a target (218, 316, 404), and at least one reflection of the ranging beam off the distal end of the optical fiber,
analyze (606) the detection signal to determine a frequency component corresponding to the at least one reflection of the ranging beam off the target and the at least one reflection of the ranging beam off the distal end of the optical fiber, and
determine (608) a distance (224) between the distal end of the optical fiber and the target based on the frequency component,
wherein the instructions, when executed by the processor, further cause the processor to generate a treatment beam (234) with the second laser source when the distance between the distal end of the optical fiber and the target is within a threshold distance.

2. The apparatus of claim 1, wherein the instructions, when executed by the processor, further cause the processor to select a mode of operation for the second laser source based on the distance between the distal end of the optical fiber and the target.

3. The apparatus of claim 1 or 2, wherein the instructions, when executed by the processor, further cause the processor to cease generation of the treatment beam with the second laser source when the distance between the distal end of the optical fiber and the target exceeds the threshold distance.

4. The apparatus of any of claims 1 to 3, wherein the instructions, when executed by the processor, further cause the processor to generate one or more of an audible, a tactile, and a visual alert when the distance between the distal end of the optical fiber and the target exceeds a threshold distance.

5. The apparatus of any of claims 1 to 4, wherein the detector is configured to generate the detection signal based on measurement of the at least one reflection of the ranging beam off the target, the at least one reflection of the ranging beam off the distal end of the optical fiber, and at least one reflection of the ranging beam off a proximal end (220) of the optical fiber.

6. The apparatus of claim 5, wherein the frequency component comprises a first frequency component and the instructions, when executed by the processor, further cause the processor to analyze the detection signal to determine second and third frequency components, the second frequency component corresponding to the at least one reflection of the ranging beam off the distal end of the optical fiber and the at least one reflection of the ranging beam off the proximal end of the optical fiber and the third frequency component corresponding to the at least one reflection of the ranging beam off the target and the at least one reflection of the ranging beam off the proximal end of the optical fiber.

7. The apparatus of claim 6, wherein the instructions, when executed by the processor, further cause the processor to determine the first frequency component corresponds to the at least one reflection of the ranging beam off the target and the at least one reflection of the ranging beam off the distal end of the optical fiber based on the first frequency component being higher than the second and third frequency components.

8. The apparatus of any of claims 1 to 7, comprising a filter configured to remove frequencies below a threshold corresponding to reflections of the ranging beam off a proximal end of the optical fiber.

9. The apparatus of any of claims 1 to 7, comprising a bandpass filter configured to remove frequencies above a first threshold and below a second threshold.

10. At least one non-transitory computer-readable medium comprising a set of instructions that, in response to being executed by a processor circuit, cause the processor circuit to:
generate (602) a ranging beam (216, 314, 408) with a first laser source, the ranging beam including a linearly changing wavelength sweep;
identify (604) a detection signal (214, 312) generated by a detector (108, 202, 302) based on measurement of a mixture of a reference signal (208), at least one reflection (410) of the ranging beam off a target (218, 316, 404), and at least one reflection of the ranging beam off a distal end (222) of an optical fiber (106, 206, 306, 402);
analyze (606) the detection signal to determine a frequency component corresponding to the at least one reflection of the ranging beam off the target and the at least one reflection of the ranging beam off the distal end of the optical fiber;
determine (608) a distance (224) between the distal end of the optical fiber and the target based on the frequency component; and
generate a treatment beam (234) with a second laser source when the distance between the distal end of the optical fiber and the target is within a threshold distance.

11. The at least one non-transitory computer-readable medium of claim 10, wherein the set of instructions, in response to execution by the processor circuit, further cause the processor circuit to generate one or more of an audible, a tactile, and a visual alert when the distance between the distal end of the optical fiber and the target exceeds the threshold distance.

12. The at least one non-transitory computer-readable medium of any of claims 10 to 11, wherein the set of instructions, in response to execution by the processor circuit, further cause the processor circuit to perform a Fourier analysis on the detection signal to determine the frequency component.

13. The at least one non-transitory computer-readable medium of any of claims 10 to 12, wherein the set of instructions, in response to execution by the processor circuit, further cause the processor circuit to remove frequencies from the detection signal above a first threshold and below a second threshold.

14. The at least one non-transitory computer-readable medium of any of claims 10 to 13, wherein the set of instructions, in response to execution by the processor circuit, further cause the processor circuit to filter out at least a portion of the detection signal based on a reflection frequency associated with a proximal end of the optical fiber.

## Patentansprüche

1. Vorrichtung, aufweisend:
eine Laserquelle (104, 204, 304) umfassend eine erste Laserquelle;
eine zweite Laserquelle;
eine optische Faser (106, 206, 306, 402) mit einem distalen Ende (222), wobei die optische Faser konfiguriert ist, Laserlicht von der Laserquelle aus dem distalen Ende hinaus zu leiten und reflektiertes Laserlicht in das distale Ende zu empfangen;
einen Detektor (108, 202, 302); und
eine Steuerung (110, 212, 310) mit einem Prozessor (804) und Speicher (810), wobei der Speicher Anweisungen aufweist, die bei Ausführung durch den Prozessor den Prozessor veranlassen:
mit der Laserquelle einen Abtaststrahl (216, 314, 408) zu erzeugen (602), wobei der Abtaststrahl eine Abtastung mit sich linear ändernder Wellenlänge aufweist,
ein Detektionssignal (214, 312) zu identifizieren (604), das von einem Detektor erzeugt wird basierend auf Messung einer Mischung aus einem Referenzsignal (208), mindestens einer Reflexion (410) des Abtaststrahls an einem Ziel (218, 316, 404), und mindestens einer Reflexion des Abtaststrahls an dem distalen Ende der optischen Faser,
das Detektionssignal zu analysieren (606), um eine Frequenzkomponente zu bestimmen, die der mindestens einen Reflexion des Abtaststrahls an dem Ziel und der mindestens einen Reflexion des Abtaststrahls an dem distalen Ende der optischen Faser entspricht, und
basierend auf der Frequenzkomponente einen Abstand (224) zwischen dem distalen Ende der optischen Faser und dem Ziel zu bestimmen (608),
wobei die Anweisungen bei Ausführung durch den Prozessor den Prozessor ferner veranlassen, einen Behandlungsstrahl (234) mit der zweiten Laserquelle zu erzeugen, wenn der Abstand zwischen dem distalen Ende der optischen Faser und dem Ziel innerhalb eines Schwellenabstands liegt.

2. Vorrichtung nach Anspruch 1, wobei die Anweisungen bei Ausführung durch den Prozessor den Prozessor ferner veranlassen, basierend auf dem Abstand zwischen dem distalen Ende der optischen Faser und dem Ziel einen Betriebsmodus für die zweite Laserquelle auszuwählen.

3. Vorrichtung nach Anspruch 1 oder 2, wobei die Anweisungen bei Ausführung durch den Prozessor den Prozessor ferner veranlassen, Erzeugung des Behandlungsstrahls mit der zweiten Laserquelle zu beenden, wenn der Abstand zwischen dem distalen Ende der optischen Faser und dem Ziel den Schwellenabstand überschreitet.

4. Vorrichtung nach irgendeinem der Ansprüche 1 bis 3, wobei die Anweisungen bei Ausführung durch den Prozessor den Prozessor ferner veranlassen, eines oder mehrere zu erzeugen aus einem hörbaren, einem taktilen, und einem visuellen Alarm, wenn der Abstand zwischen dem distalen Ende der optischen Faser und dem Ziel den Schwellenabstand überschreitet.

5. Vorrichtung nach irgendeinem der Ansprüche 1 bis 4, wobei der Detektor konfiguriert ist, das Detektionssignal zu erzeugen basierend auf Messung der mindestens einen Reflexion des Abtaststrahls an dem Ziel, der mindestens einen Reflexion des Abtaststrahls an dem distalen Ende der optischen Faser, und mindestens einer Reflexion des Abtaststrahls an einem proximalen Ende (220) der optischen Faser.

6. Vorrichtung nach Anspruch 5, wobei die Frequenzkomponente eine erste Frequenzkomponente aufweist, und die Anweisungen bei Ausführung durch den Prozessor den Prozessor ferner veranlassen, das Detektionssignal zu analysieren, um eine zweite und eine dritte Frequenzkomponente zu bestimmen, wobei die zweite Frequenzkomponente der mindestens einen Reflexion des Abtaststrahls an dem distalen Ende der optischen Faser und der mindestens einen Reflexion des Abtaststrahls an dem proximalen Ende der optischen Faser entspricht und die dritte Frequenzkomponente der mindestens einen Reflexion des Abtaststrahls an dem Ziel und der mindestens einen Reflexion des Abtaststrahls an dem proximalen Ende der optischen Faser entspricht.

7. Vorrichtung nach Anspruch 6, wobei die Anweisungen bei Ausführung durch den Prozessor den Prozessor ferner veranlassen, basierend darauf, dass die erste Frequenzkomponente höher als die zweite und die dritte Frequenzkomponente ist, zu bestimmen, dass die erste Frequenzkomponente der mindestens einen Reflexion des Abtaststrahls an dem Ziel und der mindestens einen Reflexion des Abtaststrahls an dem distalen Ende der optischen Faser entspricht.

8. Vorrichtung nach irgendeinem der Ansprüche 1 bis 7, aufweisend ein Filter, das konfiguriert ist, Frequenzen unterhalb einer Schwelle korrespondierend zu Reflexionen des Abtaststrahls an einem proximalen Ende der optischen Faser zu entfernen.

9. Vorrichtung nach irgendeinem der Ansprüche 1 bis 7, aufweisend ein Bandpassfilter, das konfiguriert ist, Frequenzen oberhalb einer ersten Schwelle und unterhalb einer zweiten Schwelle zu entfernen.

10. Mindestens ein nichtflüchtiges computerlesbares Medium mit einem Satz von Anweisungen, die als Antwort auf Ausführung durch eine Prozessorschaltung die Prozessorschaltung veranlassen:
mit einer ersten Laserquelle einen Abtaststrahl (216, 314, 408) zu erzeugen (602), wobei der Abtaststrahl eine Abtastung mit sich linear ändernder Wellenlänge aufweist,
ein Detektionssignal (214, 312) zu identifizieren (604), das von einem Detektor (108, 202, 302) erzeugt wird basierend auf Messung einer Mischung aus einem Referenzsignal (208), mindestens einer Reflexion (410) des Abtaststrahls an einem Ziel (218, 316, 404), und mindestens einer Reflexion des Abtaststrahls an einem distalen Ende (222) einer optischen Faser (106, 206, 306, 402);
das Detektionssignal zu analysieren (606), um eine Frequenzkomponente zu bestimmen, die der mindestens einen Reflexion des Abtaststrahls an dem Ziel und der mindestens einen Reflexion des Abtaststrahls an dem distalen Ende der optischen Faser entspricht;
basierend auf der Frequenzkomponente einen Abstand (224) zwischen dem distalen Ende der optischen Faser und dem Ziel zu bestimmen (608); und
mit einer zweiten Laserquelle einen Behandlungsstrahl (234) zu erzeugen, wenn der Abstand zwischen dem distalen Ende der optischen Faser und dem Ziel innerhalb eines Schwellenabstands liegt.

11. Das mindestens eine nichtflüchtige computerlesbare Medium nach Anspruch 10, wobei der Satz von Anweisungen als Antwort auf Ausführung durch die Prozessorschaltung die Prozessorschaltung ferner veranlassen, eines oder mehrere zu erzeugen aus einem hörbaren, einem taktilen, und einem visuellen Alarm, wenn der Abstand zwischen dem distalen Ende der optischen Faser und dem Ziel einen Schwellenabstand überschreitet.

12. Das mindestens eine nichtflüchtige computerlesbare Medium nach irgendeinem der Ansprüche 10 bis 11, wobei der Satz von Anweisungen als Antwort auf Ausführung durch die Prozessorschaltung die Prozessorschaltung ferner veranlassen, eine Fourier-Analyse an dem Detektionssignal durchzuführen, um die Frequenzkomponente zu bestimmen.

13. Das mindestens eine nichtflüchtige computerlesbare Medium nach irgendeinem der Ansprüche 10 bis 12, wobei der Satz von Anweisungen als Antwort auf Ausführung durch die Prozessorschaltung die Prozessorschaltung ferner veranlassen, Frequenzen oberhalb einer ersten Schwelle und unterhalb einer zweiten Schwelle aus dem Detektionssignal zu entfernen.

14. Das mindestens eine nichtflüchtige computerlesbare Medium nach irgendeinem der Ansprüche 10 bis 13, wobei der Satz von Anweisungen als Antwort auf Ausführung durch die Prozessorschaltung die Prozessorschaltung ferner veranlassen, basierend auf einer Reflexionsfrequenz, die mit einem proximalen Ende der optischen Faser assoziiert ist, mindestens einen Teil des Detektionssignals herauszufiltern.

## Revendications

1. Appareil, comprenant :
une source laser (104, 204, 304) comprenant une première source laser ;
une deuxième source laser ;
une fibre optique (106, 206, 306, 402) ayant une extrémité distale (222), la fibre optique étant configurée pour transmettre la lumière laser provenant de la source laser vers l'extérieur de l'extrémité distale et pour recevoir la lumière laser réfléchie à l'intérieur de l'extrémité distale ;
un détecteur (108, 202, 302) ; et
un contrôleur (110, 212, 310) comprenant un processeur (804) et une mémoire (810), la mémoire comprenant des instructions qui, lorsqu'elles sont exécutées par le processeur, amènent le processeur à :
générer (602) un faisceau de télémétrie (216, 314, 408) à l'aide de la source laser, le faisceau de télémétrie comprenant un balayage de longueur d'onde variant linéairement,
identifier (604) un signal de détection (214, 312) généré par un détecteur sur la base de la mesure d'un mélange d'un signal de référence (208), d'au moins une réflexion (410) du faisceau de télémétrie sur une cible (218, 316, 404), et d'au moins une réflexion du faisceau de télémétrie sur l'extrémité distale de la fibre optique,
analyser (606) le signal de détection afin de déterminer une composante de fréquence correspondant à l'au moins une réflexion du faisceau de télémétrie sur la cible et à l'au moins une réflexion du faisceau de télémétrie sur l'extrémité distale de la fibre optique, et
déterminer (608) une distance (224) entre l'extrémité distale de la fibre optique et la cible sur la base de la composante de fréquence,
les instructions,
lorsqu'elles sont exécutées par le processeur, amenant en outre le processeur à générer un faisceau de traitement (234) à l'aide de la deuxième source laser lorsque la distance entre l'extrémité distale de la fibre optique et la cible est inférieure ou égale à une distance seuil.

2. Appareil selon la revendication 1, dans lequel les instructions, lorsqu'elles sont exécutées par le processeur, amènent en outre le processeur à sélectionner un mode de fonctionnement pour la deuxième source laser sur la base de la distance entre l'extrémité distale de la fibre optique et la cible.

3. Appareil selon la revendication 1 ou 2, dans lequel les instructions, lorsqu'elles sont exécutées par le processeur, amènent en outre le processeur à arrêter la génération du faisceau de traitement à l'aide de la deuxième source laser lorsque la distance entre l'extrémité distale de la fibre optique et la cible dépasse la distance seuil.

4. Appareil selon l'une quelconque des revendications 1 à 3, dans lequel les instructions, lorsqu'elles sont exécutées par le processeur, amènent en outre le processeur à générer une ou plusieurs alertes parmi une alerte audible, une alerte tactile et une alerte visuelle, lorsque la distance entre l'extrémité distale de la fibre optique et la cible dépasse une distance seuil.

5. Appareil selon l'une quelconque des revendications 1 à 4, dans lequel le détecteur est configuré pour générer le signal de détection sur la base de la mesure de l'au moins une réflexion du faisceau de télémétrie sur la cible, de l'au moins une réflexion du faisceau de télémétrie sur l'extrémité distale de la fibre optique, et d'au moins une réflexion du faisceau de télémétrie sur une extrémité proximale (220) de la fibre optique.

6. Appareil selon la revendication 5, dans lequel la composante de fréquence comprend une première composante de fréquence et les instructions, lorsqu'elles sont exécutées par le processeur, amènent en outre le processeur à analyser le signal de détection afin de déterminer une deuxième et une troisième composantes de fréquence, la deuxième composante de fréquence correspondant à l'au moins une réflexion du faisceau de télémétrie sur l'extrémité distale de la fibre optique et à l'au moins une réflexion du faisceau de télémétrie sur l'extrémité proximale de la fibre optique, et la troisième composante de fréquence correspondant à l'au moins une réflexion du faisceau de télémétrie sur la cible et à l'au moins une réflexion du faisceau de télémétrie sur l'extrémité proximale de la fibre optique.

7. Appareil selon la revendication 6, dans lequel les instructions, lorsqu'elles sont exécutées par le processeur, amènent en outre le processeur à déterminer que la première composante de fréquence correspond à l'au moins une réflexion du faisceau de télémétrie sur la cible et l'au moins une réflexion du faisceau de télémétrie sur l'extrémité distale de la fibre optique sur la base du fait que la première composante de fréquence est supérieure à la deuxième et à la troisième composantes de fréquence.

8. Appareil selon l'une quelconque des revendications 1 à 7, comprenant un filtre configuré pour supprimer les fréquences inférieures à un seuil correspondant aux réflexions du faisceau de télémétrie sur une extrémité proximale de la fibre optique.

9. Appareil selon l'une quelconque des revendications 1 à 7, comprenant un filtre passe-bande configuré pour supprimer les fréquences supérieures à un premier seuil et inférieures à un deuxième seuil.

10. Au moins un support non transitoire lisible par ordinateur comprenant un ensemble d'instructions qui, en réponse à leur exécution par un circuit processeur, amènent le circuit processeur à :
générer (602) un faisceau de télémétrie (216, 314, 408) à l'aide d'une première source laser, le faisceau de télémétrie comprenant un balayage de longueur d'onde variant linéairement ;
identifier (604) un signal de détection (214, 312) généré par un détecteur (108, 202, 302) sur la base de la mesure d'un mélange d'un signal de référence (208), d'au moins une réflexion (410) du faisceau de télémétrie sur une cible (218, 316, 404), et d'au moins une réflexion du faisceau de télémétrie sur une extrémité distale (222) d'une fibre optique (106, 206, 306, 402) ;
analyser (606) le signal de détection afin de déterminer une composante de fréquence correspondant à l'au moins une réflexion du faisceau de télémétrie sur la cible et à l'au moins une réflexion du faisceau de télémétrie sur l'extrémité distale de la fibre optique ;
déterminer (608) une distance (224) entre l'extrémité distale de la fibre optique et la cible sur la base de la composante de fréquence ; et
générer un faisceau de traitement (234) à l'aide d'une deuxième source laser lorsque la distance entre l'extrémité distale de la fibre optique et la cible est inférieure ou égale à une distance seuil.

11. Au moins un support non transitoire lisible par ordinateur selon la revendication 10, dans lesquels l'ensemble d'instructions, en réponse à leur exécution par le circuit processeur, amènent en outre le circuit processeur à générer une ou plusieurs alertes parmi une alerte audible, une alerte tactile et une alerte visuelle, lorsque la distance entre l'extrémité distale de la fibre optique et la cible dépasse une distance seuil.

12. Au moins un support non transitoire lisible par ordinateur selon l'une quelconque des revendications 10 à 11, dans lequel l'ensemble d'instructions, en réponse à l'exécution par le circuit processeur, amènent en outre le circuit processeur à effectuer une analyse de Fourier sur le signal de détection afin de déterminer la composante de fréquence.

13. Au moins un support non transitoire lisible par ordinateur selon l'une quelconque des revendications 10 à 12, dans lequel l'ensemble d'instructions, en réponse à l'exécution par le circuit processeur, amènent en outre le circuit processeur à supprimer les fréquences du signal de détection supérieures à un premier seuil et inférieures à un deuxième seuil.

14. Au moins un support non transitoire lisible par ordinateur selon l'une quelconque des revendications 10 à 13, dans lequel l'ensemble d'instructions, en réponse à l'exécution par le circuit processeur, amènent en outre le circuit processeur à filtrer au moins une partie du signal de détection sur la base d'une fréquence de réflexion associée à une extrémité proximale de la fibre optique.
